Europäisches Patentamt

European Patent Office

Office européen des brevets

(19)

(11) Numéro de publication: **0 003 923**
**B1**

(12) **FASCICULE DE BREVET EUROPEEN**

(45) Date de publication du fascicule du brevet: **20.10.82**

(51) Int. Cl.³: **C 12 N 11/06, C 12 N 9/00**

(21) Numéro de dépôt: **79400070.3**

(22) Date de dépôt: **05.02.79**

(54) Complexes support-enzyme et leur procédé de préparation.

(30) Priorité: **16.02.78 FR 7804354**
**23.01.79 FR 7901580**

(43) Date de publication de la demande:
**05.09.79 Bulletin 79/18**

(45) Mention de la délivrance du brevet:
**20.10.82 Bulletin 82/42**

(84) Etats contractants désignés:
**BE CH DE GB IT NL SE**

(56) Documents cités:
**FR - A - 2 192 113**
**FR - A - 2 278 702**

**CHEMICAL ABSTRACTS, vol. 75, 10634x
(1971), no. 2, July 12, 1971 Columbus, Ohio,
USA E. A. CHUIKO et al.: "Interaction of
monoethanolamine with a silica surface studied
by an ir-spectroscopic method". page 322.**

(73) Titulaire: **RHONE-POULENC SPECIALITES
CHIMIQUES**
**"Les miroirs" 18, Boucle d'Alsace**
**F-92400 Courbevoie (FR)**

(72) Inventeur: **Mirabel, Bernard**
**4, rue Louis Bonin**
**F-94310 Orly (FR)**
Inventeur: **Meiller, François**
**2, rue Jeanne d'Arc**
**F-91120 Palaiseau (FR)**

(74) Mandataire: **Lepere, Michelle et al,**
**RHONE POULENC RECHERCHES Service Brevets
Chimie et Polymères 25, Quai Paul Doumer**
**F-92408 Courbevoie Cedex (FR)**

(56) Documents cités:
**CHEMICAL ABSTRACTS, vol. 69, no. 19
November 4, 1968 ref. 76654m (1978)
Columbus, Ohio, USA A. CHUIKO et al.:
"Reaction of silica gel with lactic acid", page
7153.**

Courier Press, Leamington Spa, England.

R. G. AZRAK and C. L. ANGELL, Study of
Alcohol-Silica Surface Reactions via Infrared
Spectroscopy, J. Phys. Chem., 77 (26), 3048—
52 (1973).
"Immobilised Enzymes" CRC Press, Inc. (1977).
pp. 6, 12, 13, 28—46.
J. of Chrom. 141 (1977)—291.

Le dossier contient des informations techniques
· présentées postérieurement au dépôt de la
demande et ne figurant pas dans le présent
fascicule.

Complexer support-enzyme et leur procédé de préparation

L'invention a pour objet des complexes support-enzyme stables et actifs, ainsi que leur procédé de préparation.

Pour immobiliser des enzymes, on a déjà utilisé des supports possédant des groupes réactifs avec les enzymes. C'est ainsi que dans "Immobilized Enzymes" de O. Zaborsky (1977) sont décrites des fixations d'enzymes sur des celluloses, des polysaccharides ou du verre, qui sont modifiés par différentes techniques, telles que adsorption de composés divers, greffage de silanes, pour leur apporter les groupes réactifs nécessaires. Toutefois, les complexes obtenus ne possédent pas toujours une activité enzymatique suffisante. En outre, les celluloses et polysaccharides ne possédent pas de propriétés mécaniques, ils gonflent dans l'eau et les solvants organiques et n'ont aucune tenue aux températures et pressions; par ailleurs des composés adsorbés sur le support risquent d'être éliminés au cours des applications ultérieures; quant aux silanes, ce sont des produits très particuliers de prix élevé, ne donnant pas toujours satisfaction.

On connaît également la réaction d'alcools sur des supports portant des groupes OH, telle que décrite dans Chemical Abstracts *69*, 76654m (1968), Chemical Abstracts 75, 10634x (1971), et "Study of Alcohol-Silica Surface Reactions" J. Phys. Chem. 77 (26), 3048—52 (1973). Cependant, cette réaction donne généralement des liaisons qui ne sont pas stables, car elles s'hydrolisent facilement, et aucune fixation d'enzymes n'est effectuée sur les produits obtenus.

Les complexes support-enzyme de l'invention ne présentent pas ces inconvénients. Ils sont particulièrement stables et enzymatiquement actifs. Ils sont à base de supports minéraux greffés qui possèdent de très bonnes propriétés mécaniques, sont insensibles aux solvants, températures et pressions, et dont les greffons sont des composés organiques courants.

Les complexes support-enzyme stables et enzymatiquement actifs sont constitués d'enzymes fixées par liaison covalente à des supports minéraux greffés et sont caractérisé en ce que les enzymes sont liées à au moins une fonction réactive de groupements organiques greffés aux supports par au moins une liaison ester.

Les groupements organiques sont représentés par des restes aliphatiques linéaires ou ramifiés, dont la chaine de 1 à 8 atomes de carbone peut contenir un atome d'azote et/ou un atome de soufre et/ou un groupe phényle; des restes cyclaniques; des restes aryles ou alkylaryles, dont le noyau et/ou la chaîne peuvent contenir un ou plusieurs atomes d'azote.

En tant que fonction réactive avec l'enzyme, on peut citer plus particulièrement les fonctions amine, halogène, sulfhydryle, aldéhyde, carboxyle.

Les complexes selon l'invention sont obtenus par réaction d'un support minéral insoluble possédant des groupes hydroxyle avec un composé formé d'un groupement organique possédant au moins une fonction alcool ou phénol et au moins une fonction réactive, à laquelle l'enzyme est ensuite liée.

Le support minéral mis en oeuvre doit être insoluble dans l'eau et posséder des groupes hydroxyle en surface. Il se présente sous forme de blocs, morceaux, billes, perles, objets, fils, tissus, dont la surface peut être poreuse ou non; le choix du support étant fonction de l'application du complexe. Par exemple, lorsque le complexe support-enzyme est mis en oeuvre dans une colonne fonctionnant en continu, il est avantageux que le support finement divisé ait une granulométrie comprise entre 5 $\mu$m et 5 mm. Quant à la porosité du support, elle est fonction de l'activité enzymatique du complexe; l'activité étant d'autant plus grande que la surface interne accessible est plus grande.

Le support minéral est représenté plus particulièrement par la brique; les silicates et aluminosilicates alcalins; les oxydes métalliques comme les alumines et le dioxyde de titane; les silices.

Le composé organique est un alcool ou un phénol dont les fonctions réactives avec l'enzyme: amine, halogène, sulfhydryle, aldéhyde, carboxyle, et les fonctions —OH sont liées à:

—des restes aliphatiques linéaires ou ramifiés dont la chaine de 1 à 8 atomes de carbone peut contenir un atome d'azote et/ou un atome de soufre et/ou un groupe phényle. Parmi ces composés, peuvent être cités les aminoalcools, tels que: monoéthanolamines, aminopropanols, aminométhyl-propanols, phényl-amino-propanols, aminobutanols, aminopentanols, aminométhyl-heptanols, aminoéthyl-éthanolamine, méthioninol, amino-méthyl-propanediol, tris hydroxyméthylamino-méthane, hydroxyméthylanilines, tryptophanol; les halogènoalcools, tels que: chloroéthanols, chloropropanols, chlorométhylpropanols, chlorobutanols, chlorohexanols, chloropropanediols, alcools chlorobenzylliques, bromoéthanols, bromopropanols, bromopropanediols, alcools bromobenzyliques, bromobenzhydrols, iodoéthanols; les mercaptoalcools, tels que: mercaptoéthanols, thioglycérols, dithioérythritols; les aldéhydes-alcools, tels que: glycéraldéhydes; les acides-alcools, tel que: acides hydroxypropioniques, hydroxybutyriques, hydroxyvalériques, hydroxycaproïques, hydroxyoctanoïques, hydroxyméthylbenzoïques.

—des restes cyclaniques, comme les aminocyclohexanols;

—des restes aryle ou alkylaryle, dont le noyau et/ou la chaine peuvent contenir un ou plusieurs atomes d'azote. Ce sont par exemple les aminophénols, tels que: aminophénols, amino-crésols, amino-hydroxypyridines, amino-hydroxy-pyrimidines, amino-dihydroxypyrimidines, amino-hydroxy-méthyl-pyrimidines, les halo-génophénols, tels que: chlorophénols, chloro-crésols, chlorodiméthylphénols, bromophénols, bromonaphtols, iodophénols; les mercapto-phénols; les aldéhydes-phénols, tels que hydroxybenzaldéhydes, dihydroxybenzaldé-hydes, dihydroxyméthoxybenzaldéhydes, hydroxynaphtaldéhydes; les acides-phénols, tels que: acides hydroxybenzoîques, phlorétique, hydroxymandéliques, hydroxynaphtoîques.

Le composé organique possédant au moins une fonction alcool ou phénol et au moins une fonction réactive avec l'enzyme est greffé au support minéral par réaction entre les fonctions alcool ou phénol du composé organique et les groupes hydroxyle du support, afin d'obtenir une liaison ester. Cette réaction est réalisée à une température comprise entre la température ambiante et la température d'ébullition du milieu, suivant la réactivité des produits mis en présence. L'eau formée peut rester dans le milieu sans inconvénient ou être éliminée. De préférence, on opère à ébullition, car la réaction est plus rapide et avec élimination de l'eau, ce qui permet de contrôler le temps de réaction.

Si le composé organique est liquide, pour avoir une dispersion du support, il est nécessaire d'utiliser un excès du composé organique, soit une quantité de composé organique supérieure à 40 ml pour 100 g de support.

Dans le cas où le composé organique est solide ou même liquide, il est possible d'effectuer la réaction en présence d'un solvant du composé organique. Ce solvant doit être inerte chimiquement vis-à-vis du support et du composé organique et éventuellement donner à l'ébullition un azéotrope avec l'eau formée au cours de la réaction. A titre d'exemple, peuvent être cités le diméthylformamide, le xylène, le dioxanne. La quantité de composé organique est au minimum celle qui correspond à une fonction alcool ou phénol pour un groupe hydroxyle du support et la quantité de solvant doit être suffisante pour mouiller le support minéral.

Que l'on opère ou non en présence d'un solvant, après réaction, le support greffé est séparé du milieu, lavé avec un solvant du composé organique et, le cas échéant, séché s'il doit être conservé.

Toutes les enzymes peuvent être immobilisées sur les supports, et notamment les oxydoréductases, telles que: glucose oxydase, d-amino-acide-oxydase, lactate déshydrogénase, peroxydase, catalase; les transférases, telles que: aspartate aminotransférase, aspartate acétyltransférase, hexokinase; les hydrolases, telles que: lipase, phospholipase, acétyl-cholinestérase, pectinase, phosphatase, amylase, maltase, cellulase, invertase, acylase, pepsine, papaine, rennine, trypsine, chymo-trypsine, asparaginase, uréase, arginase, ribonucléase, lactase, broméline, pénicillineamidase; les lyases, telles que: aspartase, fumarase; les isomérases, telles que: glucose isomérase, lactate racémase; les ligases, telles que: asparagine synthétase, glutamine synthétase, pyruvate carboxylase.

Suivant la fonction réactive du greffon, l'enzyme peut réagir soit directement, soit après activation du greffon selon tous procédés connus. C'est ainsi, par exemple, que: un greffon aminé peut être activé par diazotation, ou réaction avec un polyaldéhyde ou un carbodiimide; un greffon sulfhydryle peut être activé par réaction avec la 2,2'-dithiodipyridine; un greffon carboxylique peut être activé par réaction avec un carbodiimide. Cette activation est généralement effectuée par dispersion du support greffé dans une solution aqueuse d'un excès de réactif par rapport au groupe à activer, à un pH et une température compatibles avec la réaction, puis séparation et lavage du support.

La fixation de l'enzyme est effectuée suivant tous procédés connus, soit à froid en solution aqueuse tamponnée selon le pH le plus compatible avec l'enzyme, soit à chaud dans des hydrocarbures aliphatiques, cycloaliphatiques, ou aromatiques suivant le procédé du brevet français 2.278.702.

Les complexes support-enzyme obtenus sont stables et résistants aux facteurs de dénaturation, pH, température et peuvent être utilisés aussi bien en discontinu qu'en continu, sans perdre leur activité enzymatique.

Ces complexes peuvent être employés en chromatographie d'affinité, catalyse enzymatique, analytique ou industrielle, notamment dans les industries alimentaire, pharmaceutique ou phytosanitaire.

On donne ci-après, à titre indicatif, des exemples de réalisation de l'invention.

Exemple 1

50 g d'une silice en microbilles dont la granulométrie est de 100—200 $\mu$m, la surface spécifique de 25 m2/g, le diamètre moyen des pores de 1250 Å et le volume poreux de 1 ml/g sont ajoutés à 300 ml de monoéthanolamine. La dispersion est chauffée à ébullition, puis maintenue à cette température pendant 3 heures, avec élimination progressive de 150 ml d'éthanolamine et de l'eau formée. Après refroidissement, la silice est filtrée, lavée avec 300 ml d'acétone, puis séchée à l'étuve à 80°C.

Le produit obtenu contient 0,21% en poids de carbone et 0,1% en poids d'azote, déterminés par microanalyse.

La silice greffée obtenue est traitée par 250 ml d'une solution de glutaraldéhyde à 4% en poids dans du tampon phosphate 0,1 M, pH 8, sous agitation, à température ambiante, pendant 2 heures. La silice est alors filtrée, puis lavés avec 100 ml de tampon phosphate 0,1 M, pH 8.

La silice greffée traitée est ensuite lavée avec 400 ml de tampon acétate 0,05 M, pH 4,5, puis dispersée dans 250 ml de tampon acétate 0,05 M, pH 4,5 contenant en solution 4% en poids de lactase. La dispersion est maintenue sous agitation, à température ambiante pendant 15 heures.

Le complexe support-enzyme formé est séparé par filtration, puis lavé avec 250 ml de tampon acétate 0,05 M, pH 4,5.

L'activité enzymatique du complexe est déterminée par mise en contact d' 1 g de complexe avec 10 ml d'une solution de lactose à 40 g/l dans du tampon acétate 0,05 M, pH 4,5, à 55°C, et agitation pendant 10 mn. Le complexe est séparé par filtration et le glucose formé est dosé dans le filtrat.

L'activité enzymatique du complexe est de 78 U/g (l'unité U étant la quantité de complexe qui hydrolyse une micromole de lactose par minute).

L'activité enzymatique de l'enzyme en solution, avant fixation et après fixation, est déterminée en a joutant 1 ml de la solution d'enzyme à 10 ml d'une solution de lactose à 40 g/l dans du tampon acétate 0,05 M, pH 4,5, à 55°C, en maintenant sous agitati on pendant 5 mn, puis en arrêtant la réaction en plaçant le récipient dans de l'eau bouillante pendant 5 mn. Après refroidissement, le glucose formé est dosé. Par différence entre les deux activités, on obtient l'activité disparue au cours de la fixation.

Le rapport entre l'activité de l'enzyme immobilisée et l'activité disparue de la solution donne le rendement d'immobilisation de la lactase, qui est de 55%.

A titre comparatif, on répète l'exemple 1 avec un support greffé par un aminosilane, obtenu comme suit:

—50 g des mêmes billes de silice que dans l'exemple 1 sont mis en suspension dans 250 ml de xylène contenant 5 g de $\alpha$-aminopropyltriéthoxysilane. La suspension est chauffée à ébullition pendant 3 heures. Après refroidissement, la silice est séparée par filtration, lavée avec 300 ml d'acétone, puis séchée à 80°C.

Par analyse, on détermine que le produit obtenu contient 0,70% en poids de carbone et 0,18% en poids d'azote.

Après fixation de la lactase, le complexe formé a une activité enzymatique de 80 U/g et le rendement d'immobilisation est de 22%.

On constate que les deux complexes ont sensiblement la même activité, mais que pour obtenir cette activité, il faut 2,5 fois moins d'enzyme avec le support selon l'invention.

—Mise en oeuvre du complexe—

10 g de complexe de l'exemple 1 sont placés dans une colonne de 2,5 cm de diamètre, maintenue à 55°C.

On percole en continu, à raison de 200 ml/h, une solution de lactose à 40 g/l dans un tampon acétate 0,05 M, pH 4,5. Le taux d'hydrolyse du lactose en glucose, mesuré sur la solution sortant de la colonne, est de 80%.

Après 500 heures de fonctionnement en continu, le taux d'hydrolyse est toujours de 80%. Ce qui montre la bonne stabilité du complexe support-lactase de l'invention.

Exemple 2

L'exemple 1 est répété, mais il n'y a pas élimination d'une partie d'éthanolamine, ni de l'eau formée dans la préparation du support greffé, qui contient 0,2% en poids de carbone et 0,1% en poids d'azote.

On constate que l'eau restée dans le milieu n'a pas d'influence sur le greffage.

Le complexe support-lactase obtenu a une activité enzymatique de 80 U/g et le rendement d'immobilisation est de 55%.

Exemple 3

On prépare un complexe support-lactase comme dans l'exemple 1, mais avec une silice de granulométrie 200 $\mu$m-1 mm.

L'activité enzymatique du complexe est de 64 U/g et le rendement d'immobilisation de 55%.

Exemple 4

On opère comme dans l'exemple 1, mais la silice est remplacée par une alumine ayant une granulométrie de 100—200 $\mu$m, une surface spécifique de 154 m2/g, un diamètre moyen des pores de 350 Å et un volume poreux de 1,18 ml/g.

L'analyse montre que l'alumine greffée contient 1,98% en poids de carbone et 0,87% en poids d'azote.

Le complexe support-lactase obtenu a une activité enzymatique de 90 U/g et le rendement d'immobilisation est de 35%.

Exemple 5

50 g de silice ayant une granulométrie de 100 à 200 $\mu$m, une surface spécifique de 25 m2/g, un diamètre moyen des pores de 1250 Å et un volume poreux de 1 ml/g sont dispersés dans 300 ml de diméthylformamide dans lesquels sont dissous 20 g d'amino-5 pentanol-1. La dispersion est chauffée à ébullition et maintenue à cette température pendant 3 heures, avec élimination progressive de 150 ml de diméthylformamide et de l'eau formée. Après refroidissement, la silice est filtrée, lavée avec 300 ml de diméthylformamide, puis avec 150 ml d'acétone et enfin séchée à 80°C.

L'analyse montre que le produit obtenu contient 0,35% en poids de carbone et 0,09% en poids d'azote.

La silice greffée obtenue est traitée par 250 ml d'une solution de glutaraldéhyde à 4% en poids dans du tampon phosphate 0,1 M, pH 8, sous agitation, à température ambiante, pendant 2 heures. La silice est alors filtrée, puis lavée avec 100 ml de tampon phosphate 0,1 M, pH 8.

La silice greffée traitée est ensuite lavée avec 400 ml de tampon acétate 0,05 M, pH 4,5, puis dispersée dans 250 ml de tampon acétate 0,05 M, pH 4,5 contenant en solution 4% en poids de lactase. La dispersion est maintenue sous agitation, à température ambiante, pendant 15 heures.

Le complexe support-enzyme formé est séparé par filtration, puis lavé avec 250 ml de tampon acétate 0,05 M, pH 4,5.

Il possède une activité enzymatique de 40 U/g et le rendement d'immobilisation est de 45%.

Exemple 6

Pour la préparation du support greffé, on opère comme dans l'exemple 1, avec une silice ayant une granulométrie de 100 à 200 $\mu$m, une surface spécifique de 100 m2/g, un diamètre moyen des pores de 300 Å et une volume poreux de 1 ml/g.

La silice greffée obtenue contient 0,95% en poids de carbone et 0,38% en poids d'azote.

Par comparaison avec l'exemple 1, on constate que la mise en oeuvre d'une silice de plus grande surface spécifique permet d'avoir un plus grand nombre de greffons.

La silice greffée obtenue est traitée par 250 ml de solution de glutaraldéhyde à 4% en poids dans du tampon phosphate 0,1 M, pH 8, sous agitation, à température ambiante, pendant 2 heures. Après filtration, la silice greffée modifiée obtenue est lavée avec 100 ml de tampon phosphate 0,1 M, pH 8.

10 g de la silice sont ensuite lavés avec 400 ml de tampon phosphate 0,02 M, pH 7, puis dispersés dans 500 ml de tampon phosphate 0,02 M, pH 7 contenant en solution 1% en poids de pénicilline amidase. La dispersion est maintenue sous agitation, à température ambiante pendant 15 heures. Le complexe support-pénicilline amidase formé est séparé par filtration, puis lavé avec 50 ml de tampon phosphate 0,02 M, pH 7.

L'activité enzymatique du complexe support-pénicilline amidase est déterminée par mise en contact de 0,5 g de complexe avec 40 ml d'une solution de Péni G à 20 g/l dans du tampon phosphate 0,1 M, pH 7,5, à 30°C, pendant 30 minutes. Le complexe est alors séparé par filtration et le 6 A.PA formé dosé dans le filtrat.

L'activité enzymatique du complexe est de 50 U/g (l'unité U est la quantité de complexe qui hydrolyse une micromole de Péni G par minute).

L'activité enzymatique de l'enzyme en solution, avant et après fixation, est déterminée en ajoutant 1 ml de la solution d'enzyme à 10 ml d'une solution de Péni G à 20 g/l dans du tampon phosphate 0,1 M, pH 7,6, en maintenant sous agitation à 30°C pendant 30 minutes, puis en dosant le 6.A.PA formé.

Le rendement d'immobilisation de l'enzyme est de 70%.

—Mise en oeuvre du complexe—

1 g du complexe est placé dans une colonne de 1 cm de diamètre.

On percole en continu et à temperature ambiante, à raison de 50 ml/h, une solution de Péni G à 20 g/l dans du tampon phosphate 0,1 M, pH 7,6.

Le taux d'hydrolyse de la Péni G en 6 A.PA, mesuré sur la solution sortant de la colonne, est de 40%.

Après 300 heures de fonctionnement, le taux d'hydrolyse est toujours de 40%. Ce qui montre la bonne stabilité du complexe support pénicilline amidase.

Exemple 7

50 g de silice ayant une granulométrie de 100 à 200 $\mu$m, une surface spécifique de 25 m2/g, un diamètre moyen des pores de 1250 Å et un volume poreux de 1 ml/g sont dispersés dans 300 ml de diméthylformamide dans lesquels sont dissous 20 g de p-aminophénol.

La dispersion est chauffée à ébullition et maintenue à cette température pendant 3 heures, avec élimination progressive de 150 ml de diméthylformamide et de l'eau formée. Après refroidissement, la silice est filtrée, lavée avec 300 ml de diméthylformamide, puis avec 150 ml d'acétone et enfin séchée à 80°C. L'analyse montre que le produit obtenu contient 0,31% en poids de carbone et 0,06% en poids d'azote.

La silice greffée est traitée par 250 ml de solution de glutaraldéhyde à 4% en poids dans du tampon phosphate 0,1 M, pH 8, sous agitation, à température ambiante, pendant 2 heures; puis filtrée et enfin lavée avec 100 ml de tampon phosphate 0,1 M, pH 8.

Un complexe support-pénicilline amidase est préparé comme dans l'exemple 6. Il possède une activité enzymatique de 16 U/g et le rendement d'immobilisation est de 65%.

Exemple 8

10 g de la même silice greffée que celle de l'exemple 7 sont traités par 200 ml d'acide chlorhydrique N/10 et 100 ml de NaNO$_2$ M/10, sous agitation, à 0°C, pendant 3 heures.

Après filtration et lavage avec 500 ml d'eau froide, la silice greffée diazotée est dispersée dans 50 ml d'une solution de lactase à 4% en poids dans du tampon acétate 0,05 M, pH 4,5. La dispersion est maintenue sous agitation, à 4°C, pendant 3 heures. Le complexe support-lactase formé est séparé par filtration, puis lavé avec le même tampon acétate.

L'activité enzymatique du complexe obtenu est de 71 U/g et le rendement d'immobilisation de 20%.

Exemple 9

10 g de la même silice greffée diazotée que celle de l'exemple 8 sont dispersés dans 200 ml d'une solution d'acylase à 1% en poids dans du tampon TRIS 0,1 M, pH 8. La dispersion est maintenue sous agitation, à 4°C, pendant une nuit. Le complexe support-acylase formé est filtré, puis lavé avec le même tampon TRIS.

L'activité enzymatique du complexe est déterminée par mise en contact de 500 mg de complexe avec 50 ml d'une solution de N-acétyl-DL-méthionine à 0,4% en poids dans du tampon TRIS 0,1 M, pH 8 contenant $5.10^{-4}$ M de chlorure de cobalt, à 37°C et agitation pendant 30 mn. Le complexe est filtré et la L-méthionine formée est dosée dans le filtrat.

L'activité enzymatique du complexe est de 10 U/g (l'unité U étant la quantité de complexe qui transforme une micromole de N-acétyl-DL-méthionine par minute).

L'activité enzymatique de l'acylase en solution, avant et après fixation, est déterminée en a joutant 0,1 ml de la solution d'enzyme à 5 ml d'une solution de N-acétyl-DL-méthionine à 0,4% en poids dans du tampon TRIS 0,1 M, pH 8 contenant $5.10^{-4}$ M de chlorure de cobalt. Le mélange est maintenu 30 mn à 37°C, puis la réaction est arrêtée en plongeant le récipient dans l'eau bouillante pendant 3 mn. Après refroidissement, la L-méthionine formée est dosée.

Le rendement d'immobilisation de l'acylase, c'est-à-dire le rapport entre l'activité du complexe et l'activité disparue, est de 50%.

Exemple 10

Un complexe support-acylase est préparé de la même façon que dans l'exemple 9, mais avec une silice de granulométrie 30—100 $\mu$m.

L'activité enzymatique du complexe est de 10,5 U/g et le rendement d'immobilisation de 50%.

—Mise en oeuvre du complexe—

30 g de complexe sont placés dans une colonne de 1 cm de diamètre et 90 cm de hauteur.

On fait passer dans la colonne, à température ambiante, 300 ml de tampon TRIS 0,1 M, pH 8, en 2 heures, puis on injecte 2 ml du même tampon contenant 10 $\mu$moles de DL-méthionine et enfin on fait passer du tampon TRIS 0,1 M, pH 8, à raison de 10 ml/h.

La spectrométrie UV du liquide sortant de la colonne montre qu'il y a séparation de la D-méthionine et de la L-méthionine.

On recueille successivement une fraction de 3 ml contenant la D-méthionine, une fraction de 4 ml contenant un mélange de D- et L-méthionine et une fraction de 3 ml contenant la L-méthionine.

Exemple 11

50 g de billes de silice, dont la granulométrie est de 100 à 200 $\mu$m, la surface spécifique de 23 m2/g, le diamètre moyen des pores de 1330 Å et le volume poreux de 0,96 ml/g, sont ajoutés à 200 ml de xylène contenant 30 g de chloro-2 éthanol. On chauffe à ébullition, puis maintient cette température pendant 4 h. Après refroidissement, la silice est filtrée, lavée avec 300 ml d'acétone, puis séchée à l'étuve à 80°C.

Par microanalyse, on détermine que le produit obtenu contient 0,16% en poids de carbone et 0,20% en poids de chlore.

La silice greffée obtenue est dispersée dans 400 ml d'une solution à 1% en poids d'uréase dans un tampon phosphate 0,1 M, pH 6,5, puis laissée sous agitation, à température ambiante, pendant 20 heures. Le complexe support-uréase formé est filtré, puis lavé avec 250 ml de tampon phosphate 0,1 M, pH 6,5.

L'activité enzymatique du complexe est déterminée par mise en contact de 100 mg de complexe avec 20 ml d'une solution d'urée à 30 g/l dans du tampon phosphate 0,1 M, pH 7, à 37°C et agitation pendant 3 mn. Le complexe est alors séparé par filtration et l'ammoniac formé neutralisé par addition de 20 ml d'acide chlorhydrique 0,1 N à la solution séparée. L'excès d'acide est dosé par de la soude 0,1 N.

L'activité enzymatique du complexe est de 1500 U/g (l'unité U étant la quantité de complexe qui libère 1 micromole d'ammoniac par mn).

L'activité enzymatique de l'uréase en solution, avant et après fixation, est déterminée en ajoutant 0,5 ml de la solution d'enzyme à 4 ml d'une solution d'urée à 30 g/l dans du tampon phosphate 0,1 M, pH 7, à 37°C, an maintenant sous agitation pendant 3 mn, puis en arrêtant la réaction par addition de 5 ml d'acide chlorhydrique 0,1 N. L'excès d'acide est ensuite dosé par de la soude 0,1 N. Par différence entre les deux activités, on obtient l'activité disparue au cours de la fixation de l'enzyme.

Le rapport entre l'activité de l'enzyme immobilisée et l'activité disparue de la solution donne le rendement d'immobilisation de l'uréase, qui est de 60%.

Exemple 12

5 g d'une silice greffée, identique à celle de l'exemple 11, sont dispersés dans 50 ml de tampon phosphate 0,05 M, pH 7 contenant en solution 100 mg de catalase, puis la dispersion obtenue est maintenue 4 h, sous agitation, à température ambiante. Le complexe support-catalase formé est filtré, puis lavé avec 50 ml de tampon phosphate 0,05 M, pH 7.

L'activité enzymatique du complexe est déterminée comme suit: 10 mg du complexe sont ajoutés à 5 ml d'une solution d'eau oxygénée 0,02 M dans du tampon phosphate 0,05 M, pH 7, puis maintenus en dispersion par agitation, pendant 2 mn, à 25°C. La réaction de décomposition de l'eau oxygénée est arrêtée par addition de 3 gouttes d'acide sulfurique concentré et l'excès d'eau oxygénée dosé par une solution aqueuse de permanganate de potassium $5.10^{-3}$ M.

L'activité du complexe est de 2280 U/g (l'unité U est la quantité de complexe qui décompose 1 micromole d'eau oxygénée par minute).

L'activité de l'enzyme en solution est également déterminée avant et après fixation 0,2 ml de la solution d'enzyme sont ajoutés à 5 ml d'une solution d'eau oxygénée 0,02 M dans du tampon phosphate 0,05 M, pH 7, puis le mélange est agité 2 minutes à 25°C. 3 gouttes d'acide sulfurique concentré sont alors ajoutées pour arrêter la réaction et l'eau oxygénée en excès est dosée par une solution de permanganate de potassium $5.10^{-3}$ M.

Le rendement d'immobilisation de la catalase, c'est-à-dire le rapport entre l'activité du complexe et l'activité disparue, est de 11%.

Exemple 13

50 g de billes de silice, dont la granulométrie est de 100—200 $\mu$m, la surface spécifique de 23 m2/g, le diamètre moyen des pores de 1330 Å, et le volume poreux de 0,96 ml/g, sont ajoutés, à 250 ml de diméthylformamide contenant 15 g de 4-hydroxybenzaldéhyde. La dispersion obtenue est chauffée à l'ébullition, puis maintenue à cette température pendant 4 heures. Après refroidissement, la silice est filtrée, lavée avec 250 ml de diméthylformamide, puis 150 ml d'acétone et enfin séchée à l'étuve à 80°C.

Par microanalyse, on détermine que le produit obtenu contient 0,20% en poids de carbone.

Sur la silice greffée, on immobilise de l'uréase, ainsi qu'il est décrit dans l'exemple 11.

Le complexe support-uréase a une activité enzymatique de 1400 U/g et le rendement d'immobilisation est de 55%.

Exemple 14

Sur une silice greffée, identique à celle de l'exemple 13, on immobilise de la catalase de la même façon que dans l'exemple 12.

Le complexe support-catalase obtenu a une activité enzymatique de 2250 U/g et le rendement d'immobilisation est de 10%.

Exemple 15

5 g d'une silice greffée, identique à celle de l'exemple 13, sont dispersés dans 200 ml d'une solution de trypsine à 1% dans du tampon TRIS 0,1 M, pH 7,8. La dispersion obtenue est ensuite maintenue pendant 4 heures sous agitation, à température ambiante. Le complexe support-trypsine formé est filtré, puis lavé avec 50 ml de tampon TRIS 0,1 M, pH 7,8.

L'activité enzymatique du complexe est déterminée comme suit: 10 mg de complexe sont mis en contact avec 10 ml de tampon TRIS 0,2 M, pH 7,8 contenant 25 mmole/l de chlorure de calcium et avec 0,2 ml d'une solution à 12 mmole/l de benzoylarginine p-nitranilide, à 25°C, sous agitation, pendant 2 minutes. Le complexe est filtré et la p-nitraniline formée et contenue dans le filtrat est dosée par chlorimétrie.

L'activité enzymatique du complexe est de 13,8 U/g (l'unité U étant la quantité de complexe qui hydrolyse 1 micromole de benzoylarginine p-nitranilide par minute, à 25°C et a pH 7,8).

L'activité enzymatique de la trypsine en solution avant et après fixation, est déterminée en ajoutant 0,2 ml de solution d'enzyme dans du tampon TRIS 0,1 M, pH 7,8 à 2 ml de tampon TRIS 0,2 M, pH 7,8 contenant 22 mmole/l de chlorure de calcium, auquel on a ajouté 0,2 ml de solution à 12 mmole/l de benzoylarginine p-nitranilide; maintien de la solution obtenue à 25°C, pendant 15 mn, puis dosage de la paranitraniline formée.

Le rapport entre l'activité de l'enzyme immobilisée et l'activité disparue de la solution donne le rendement d'immobilisation de la trypsine qui est de 78%.

Exemple 16

50 g de la même silice greffée que celle de l'exemple 13 sont dispersés dans 250 ml de tampon acétate 0,05 M, pH 4,5 contenant en solution 4% en poids de lactase. La dispersion est maintenue sous agitation, à température ambiante, pendant 15 heures. Le complexe support-enzyme formé est séparé par filtration, puis lavé avec 250 ml de tampon acétate 0,05 M, pH 4,5.

L'activité enzymatique et le rendement d'immobilisation, déterminés comme dans l'exemple 1, sont respectivement de 85 U/g et 45%.

—Mise en oeuvre du complexe—

5 g de complexe sont placés dans une colonne de 2,5 cm de diamètre.

On percole une solution de glutaraldéhyde à 2% en poids dans du tampon phosphate 0,1 M, pH 8, à raison de 100 ml/h, pendant 3 h, à température ambiante, puis on fait passer 100 ml de tampon phosphate en 1 h. Ensuite, on percole en continu, à raison de 100 ml/h, une solution de lactose à 40 g/l dans du tampon acétate 0,05 M, pH 4,5, à 55°C.

Le taux d'hydrolyse du lactose en glucose, mesuré sur la solution sortant de la colonne, est de 69%.

Après 200 heures de fonctionnement en continu, le taux d'hydrolyse est inchangé. Ce qui montre la bonne stabilité du complexe support-lactase.

Exemple 17

30 g de billes de silice, dont la granulométrie est de 100—200 $\mu$m, la surface spécifique de 23 m2/g, le diamètre moyen des pores de 1330 Å, et le volume poreux de 0,96 ml/g, sont ajoutés à 100 ml de mercaptoéthanol. La dispersion obtenue est chauffée à l'ébullition, puis maintenue à cette température pendant 4 heures. Après refroidissement, la silice est filtrée, lavée avec 200 ml d'acétone, puis séchée à 80°C.

Par microanalyse, on détermine que le produit obtenu contient 0,25% en poids de carbone et 0,32% en poids de soufre.

La silice greffée obtenue est lavée par 250 ml d'une solution de chlorure de sodium 0,3 M dans du tampon TRIS 0,1 M, pH 8 contenant 1 mmole d'EDTA, à température ambiante, puis la silice est filtrée et ensuite mise en contact et agitée, à température ambiante, pendant 30 mn, avec 500 ml du même tampon TRIS/NaCl/EDTA contenant 30 mmole de 2,2′-dithiodipyridine. La silice est ensuite filtrée, puis lavée avec 250 ml de tampon phosphate 0,1 M, pH 7.

La silice greffée est alors dispersée dans 250 ml d'une solution à 1% en poids d'uréase dans du tampon phosphate 0,1 M, pH 6,5, puis laissée sous agitation, à température ambiante, pendant 20 heures. Le complexe support-uréase formé est filtré, puis lavé avec 150 ml de tampon phosphate 0,1 M, pH 6,5.

L'activité enzymatique du complexe et le rendement d'immobilisation, déterminés comme dans l'exemple 11, sont respectivement de 1500 U/g et de 52%.

Exemple 18

30 g de billes de silice de granulométrie 100—200 μm, surface spécifique 23 m2/g, diamètre moyen des pores 1330 Å et volume poreux 0,96 ml/g sont ajoutés à 100 ml de dioxanne contenant 25 ml d'acide hydroxy-3-propionique. La dispersion obtenue est chauffée à l'ébullition, puis maintenue à cette température pendant 4 heures. Après refroidissement, la silice est filtrée, lavée avec 200 ml d'acétone, puis séchée à l'étuve à 80°C.

Le produit obtenu contient 0,10% en poids de carbone.

La silice greffée obtenue est dispersée dans 300 ml de tampon phosphate 0,05 M, pH 5,5 contenant en solution 600 ml de catalase.

La dispersion agitée est maintenue 4 heures à température ambiante. Le complexe support-catalase formé est filtré, puis lavé avec 250 ml de tampon phosphate 0,05 M, pH 5,5.

L'activité enzymatique du complexe est de 1500 U/g, et la rendement d'immobilisation est de 10%.

Exemple 19

30 g de la même silice greffée et séchée que celle de l'exemple 18 sont dispersés dans 300 ml de tampon acétate 0,05 M, pH 4,5 contenant en solution 3 g de 1-cyclohexyl 3-(2-morpholinoéthyl)carbodiimide métho-p-toluène sulfonate. La dispersion est maintenue 3 heures à température ambiante, sous agitation. La silice est ensuite filtrée, puis lavée avec 500 ml d'eau distillée.

Sur le produit obtenu, on fixe de la trypsine de la même façon que dans l'exemple 15.

Le complexe formé a une activité enzymatique de 13,8 U/g et le rendement d'immobilisation est de 79%.

**Revendications**

1) Complexes support-enzyme constitués d'enzymes fixées par liaison covalente à des supports minéraux greffés et caractérisés en ce que les enzymes sont liées à au moins une fonction réactive représentée par les fonctions amine, halogène, sulfhydryle, aldéhyde, carboxyle de groupements organiques représentés par des restes aliphatiques linéaires ou ramifiés, dont la chaine de 1 à 8 atomes de carbone peut contenir un atome d'azote et/ou un atome de soufre et/ou un groupe phényle; des restes cyclaniques; des restes aryles ou alkylaryles, dont le noyau et/ou la chaine peuvent contenir un ou plusieurs atomes d'azote, lesdits groupements organiques étant greffés aux supports par au moins une liaison ester.

2) Procédé d'obtention des complexes selon la revendication 1, par réaction entre un support minéral insoluble possédant des groupes hydroxyle avec un composé organique possédant au moins une fonction alcool et au moins une fonction réactive, à laquelle l'enzyme est ensuite fixée, caractérisé en ce que les fonctions alcool ou phénol et les fonctions réactives, amine, halogène, sulfhydryle, aldéhyde, carboxyle, sont liées à des groupements organiques représentés par des restes aliphatiques linéaires ou ramifiés, dont la chaine de 1 à 8 atomes de carbone peut contenir un atome d'azote et/ou un atome de soufre et/ou un groupe phényle; des restes cyclaniques; des restes aryles ou alkylaryles, dont le noyau et/ou la chaine peuvent contenir un ou plusieurs atomes d'azote.

3) Procédé selon la revendication 2, caractérisé en ce que le composé organique est un aminoalcool, un halogénoalcool, un mercapto-alcool, un aldéhyde-alcool, un acide-alcool, un amino-phénol, un halogénophénol, un mercaptophénol, un aldéhyde-phénol, ou un acidephénol.

4) Procédé selon la revendication 2, caractérisé en ce que, suivant la fonction réactive du composé organique, l'enzyme peut réagir soit directement, soit après activation de ladite fonction.

**Patentansprüche**

1. Träger-Enzym Komplexe bestehend aus Enzymen, die durch kovalente Bindung an gepfropfte mineralische Träger gebunden sind, dadurch gekennzeichnet, daß die Enzyme über mindestens eine reaktionsfähige Gruppe gebunden sind ausgewählt aus den Gruppen Amino, Halogen, Sulfhydryl, Aldehyd, Carboxyl jeweils gebunden an organische Gruppen bestehend aus linearen oder verzweigten aliphatischen Resten, deren Kette mit 1 bis 8 Kohlenstoffatomen ein Stickstoffatom und/oder ein Schwefelatom und/oder eine Phenylgruppe aufweisen kann, cycloaliphatischen Resten, Arylresten oder Alkarylresten, deren Kern und/oder

Ketto ein oder mehrere Stickstoffatome enthalten kann, wobei die organischen Gruppen mit Hilfe mindestens einer Esterbindung an die Träger gebunden sind.

2. Verfahren zur Herstellung der Komplexe nach Anspruch 1, mittels Reaktion zwischen einem unlöslichen mineralischen Träger der Hydroxylgruppen aufweist, mit einer organischen Verbindung, die mindestens eine alkoholische Gruppe und mindestens eine reaktionsfähige Gruppe enthält über die dann das Enzym gebunden wird, dadurch gekennzeichnet, daß die alkoholischen oder phenolischen Gruppen und die reaktionsfähigen Gruppen, Amino, Halogen, Sulfhydryl, Aldehyd, Carboxyl an organische Gruppen gebunden sind, die dargestellt werden von linearen oder verzweigten aliphatischen Resten, deren Kette mit 1 bis 8 Kohlenstoffatomen ein Stickstoffatom und/oder ein Schwefelatom und/oder eine Phenylgruppe aufweisen kann, cycloaliphatischen Resten, Arylresten oder Alkarylresten deren Kern und/oder Kette ein oder mehrere Stickstoffatome aufweisen können.

3. Verfahren nach Anspruch 2, dadurch gekennzeichnet, daß die organische Verbindung ein Aminoalkohol, ein Halogenalkohol, ein Mercaptoalkohol, ein Aldehydalkohol, eine Hydroxysäure, ein Aminophenol, ein Halogenphenol, ein Mercaptophenol, ein eine Aldehydgruppe enthaltendes Phenol oder ein eine Säuregruppe enthaltendes Phenol ist.

4. Verfahren nach Anspruch 2, dadurch gekennzeichnet, daß je nach der reaktionsfähigen Gruppe der organischen Verbindung das Enzym entweder unmittelbar oder nach Aktivierung dieser Gruppe gebunden wird.

## Claims

1. Carrier-enzyme complexes comprising enzymes that are fixed by covalent bonding to grafted inorganic carriers and characterised in that the enzymes are joined to at least one reactive function represented by the amino, halogen, sulphydryl, aldehyde and carboxyl functions of organic groups represented by aliphatic straight or branched residues having a chain of from 1 to 8 carbon atoms which may contain a nitrogen atom and/or a sulphur atom and/or a phenyl group; cyclane residues; or aryl or alkylaryl residues in which the ring and/or chain may contain 1 or more nitrogen atoms, said organic groups being grafted to the carriers by at least one ester bond.

2. A method of obtaining complexes according to claim 1 by reaction between an insoluble inorganic carrier having hydroxyl groups with an organic compound having at least one alcohol function and at least one reactive function, to which the enzyme is then fixed, characterised in that the alcohol or phenol functions and the reactive functions, amino, halogen, sulphydryl, aldehyde or carboxyl, are joined to organic groups represented by aliphatic straight or branched residues having a chain of from 1 to 8 carbon atoms that may contain a nitrogen atom and/or a sulphur atom and/or a phenyl group; cyclane residues; or aryl or alkylaryl residues in which the ring and/or the chain may contain 1 or more nitrogen atoms.

3. A method according to claim 2 characterised in that the organic compound is an amino alcohol, a haloalcohol, a mercaptoalcohol, an alcohol-aldehyde, an alcohol-acid, an aminophenol, a halophenol, a mercaptophenol, a phenol-aldehyde or a phenol-acid.

4. A method according to claim 2 characterised in that, depending on the reactive function of the organic compound, the enzyme may react either directly or after activation of said function.